# EUROPEAN PATENT SPECIFICATION

(11) **EP 1 441 799 B1**
(45) Date of publication and mention of the grant of the patent: **11.10.2006**
(21) Application number: 02742178.3
(22) Date of filing: 17.06.2002
(51) Int. Cl.: A61F 11/00, A61M 31/00

(54) **DEVICE FOR DELIVERING MICRODOSES OF AGENT TO THE EAR**
VORRICHTUNG ZUR ABGABE VON MIKRODOSEN EINES MEDIKAMENTS IN DAS OHR
DISPOSITIF D'ADMINISTRATION DE MICRODOSES D'AGENTS DANS L'OREILLE

(30) Priority: 18.06.2001 US 299333 P
(43) Date of publication of application: 04.08.2004
(73) Proprietor: Durect Corporation, Cupertino, CA 95014 (US)
(72) Inventor: ARENBERG, Michael, H., Los Gatos, CA 95032 (US); RAMPERSAUD, Charles, Cupertino, CA 95014 (US); GILLIS, Edward, M., Cupertino, CA 95014 (US); YUM, Su, Il, Los Altos, CA 94024 (US); THEEUWES, Felix, Los Altos Hills, CA 94022 (US)
(74) Representative: Ebner von Eschenbach, Jennifer
(86) International application number: PCT/US2002/019290
(87) International publication number: WO 2002/102278

(56) References cited:
- WO-A-00/04854
- WO-A-00/33775
- WO-A-95/10984
- WO-A-98/56434
- US-A- 5 364 343
- US-A- 5 954 682
- US-A- 6 045 528

## Description

### FIELD OF THE INVENTION

This invention is in the field of devices and methods of delivering therapeutic agents to the ear.

### BACKGROUND OF THE INVENTION

Clinically important inner ear diseases, such as Meniere's disease, autoimmune or allergic inner ear disease, tinnitus, labarynthitis and various forms of hearing loss such as sudden viral deafness, can be treated with systemic medications, but this frequently requires delivery of relatively high concentrations of the drug, with consequent undesirable systemic effects. In addition, systemic treatments do not allow for unilateral delivery (i.e., delivery of a drug to only one ear). Another approach is the direct administration of therapeutic agents to various ear tissues.

The delivery of therapeutic agents in a controlled and effective manner is considerably more difficult with respect to tissue structures of the inner ear (e.g. those portions of the ear surrounded by the otic capsule bone and contained within the temporal bone which is the most dense bone tissue in the entire human body). The same situation exists with respect to tissue materials that lead into the inner ear (e.g. the round window membrane). The inner ear tissues are of minimal size and only readily accessible through microsurgical procedures. In order to treat various diseases and conditions associated with inner ear tissues, the delivery of medicines to such structures is often of primary importance.

Some compounds which are particularly useful may also be ototoxic, such as gentamycin, which is commonly delivered to the round window niche of the inner ear to treat Meniere's disease. Thus, delivery of the minimal amount required for efficacy, while avoiding cytotoxicity, is of paramount importance.

Various devices for delivery of therapeutic agents to the middle ear, inner ear, and middle-inner ear interface tissues include those described in U.S. Pat. Nos. 5,421,818; 5,474,529, 5,476,446 and 6,045,528 (the "IntraEAR®" patents) all to Arenberg, and all expressly incorporated herein by reference. Each of these patents discloses a medical treatment apparatus designed to deliver fluid materials to internal ear structures.

U.S. Pat. No. 5,421,818 describes a treatment system which includes a tubular stem attached to a reservoir portion with an internal cavity designed to retain a supply of therapeutic fluid compositions therein. The side wall of the reservoir portion further comprises fluid transfer means (e.g. pores or a semi-permeable membrane). Contact between the fluid transfer means and the round window membrane in a patient allows fluid materials to be delivered on-demand to the round window membrane, followed by diffusion of the fluid materials through the membrane into the inner ear.

U.S. Pat. No. 5,474,529 involves a therapeutic treatment apparatus with a plurality of reservoir portions (e.g. a first and a second reservoir portion in a preferred embodiment) which are connected to multiple tubular stems that are designed for implantation into the endolymphatic sac and duct using standard surgical techniques.

U.S. Pat. No. 5,476,446 discloses a therapeutic treatment apparatus that includes a reservoir portion for retaining liquid medicine materials therein, a first tubular stem on one side of the reservoir portion, and a second tubular stem on the opposite side of the reservoir portion. The second stem is designed to reside within the external auditory canal of a patient lateral to the ear drum, while the first stem is sized for placement within an opening formed in the stapes footplate/annular ligament so that medicine materials in fluid form can be delivered into the inner ear from the reservoir portion (which resides in the middle ear cavity medial to the ear drum).

Finally, U.S. Pat. No. 6,045,528 discloses an apparatus for transferring fluids into and out of the inner ear through the round window membrane that includes a cover member sized for placement over the round window niche. Fluid delivery and fluid extraction conduits are provided which are operatively connected to the cover member so that fluids can pass therethrough.

Another patent of interest is U.S. Patent number 6,120,484 (the "microwick" patent) to Silverstein that is directed to an otological implant for delivery of medicament and a method of using the implant. The implant includes a wick inserted through an aperture in a membrane. One end of the wick is in contact with the treatment site and the other end is readily accessible. The wick is made of a material that will convey medication from one end of the wick to the other end of the wick by capillary action so that the medication is delivered to the treatment site.

Yet another treatment method of interest is the use of "gelfoam" containing gentamycin. (Silverstein, H. et al, Otolaryngol Head Neck Surg 1999 May;120(5):649-55).

Although such therapy has met with success, it is approached with considerable caution because of the well-recorded risk of therapy-associated hearing loss (Federspil, P. Adv Otorhinolaryngol 1981;27:144-58) and vestibular function loss. Gentamycin is known to be ototoxic when too much is delivered to the inner ear, but it is known that very small amounts of drug are sufficient to be effective, with much less of the concomitant ototoxicity.

Many of the current methods, such as the microwick and the Gelfoam methods, allow for only semi-quantitative control of dosage, at best. With these methods, both total amount of drug delivered and rate of drug delivered varies in the milligram range). Imprecision of dosing is inherent in the design of the current treatment methods and is exacerbated by variability in clinical technique used to insert the drug delivery devices.

There is a need, therefore, for a device and method that allows delivery of a dose of therapeutic agent that is sufficiently large to treat an inner-ear disease, and is sufficiently small to avoid severe ototoxicity. There is a need for a device or method that has the ability to reproducibly and consistently delivery a given dose of therapeutic agent; the ability to reduce the variability and unpredictability of the total amount of therapeutic agent delivered; and the advantage of reducing the inconsistency of the amount of drug delivered due to variability in surgical techniques used to implant a drug-delivery device such as a catheter. There is also a need for such a device, having all the above advantages, that also is a self-contained device and that may be discretely implanted in the ear. Treatments devices and methods prior to the present disclosure do not successfully address this long-felt problem. The present invention addresses these needs, and provides related advantages as well.

A device as defined in the preamble of claim 1 is disclosed in international Patent Application WO 00/33775.

### SUMMARY OF THE INVENTION

The present invention provides devices for delivery of very low volumes and/or dosages of therapeutic agent to the inner ear. The device is a self-contained unit that reproducibly and consistently delivers a dose of therapeutic agent that is therapeutically effective, yet below the ototoxic threshold for that agent.

### BRIEF DESCRIPTIONS OF THE DRAWINGS

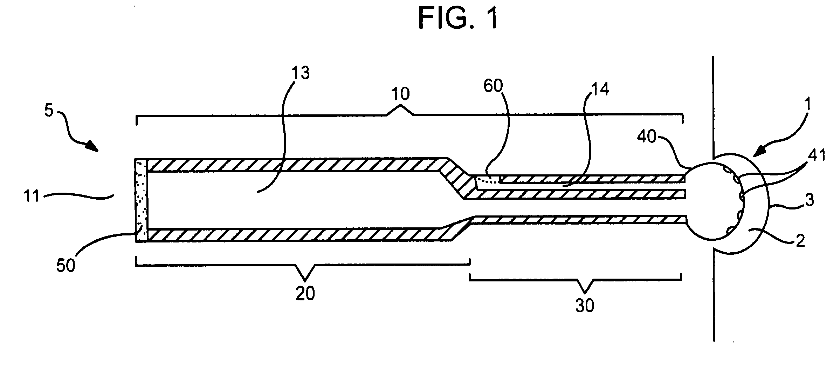

Figure 1 depicts an exemplary embodiment of a device of the invention.

### DEFINITIONS

The terms "subject," "individual," and "patient," used interchangeably herein, refer to any subject, generally a mammal (*e.g.*, human, canine, feline, ungulate, equine, bovine, rodent, *etc.*)*,* in which delivery to or into the inner ear is desired.

The term "therapeutically effective amount" is meant an amount of a therapeutic agent, or a rate of delivery of a therapeutic agent, effective to facilitate a desired therapeutic effect.

As used herein, the terms "treatment", "treating", and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment", as used herein, covers any treatment of a disease in a mammal, particularly in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

The terms "bioerodable" and biodegradable" are used interchangeably herein to refer to a material that is dissolvable in physiological conditions by physiological enzymes and/or chemical conditions.

The terms " therapeutic agent formulation," and "formulation," used interchangeably herein, are meant to encompass any substance suitable for delivery to an inner ear of a subject, which substances can include pharmaceutically active drugs, as well as biocompatible substances that do not exhibit a pharmaceutical activity in and of themselves, but that provide for a desired effect at a treatment site, *e.g.*, to flush or irrigate a treatment site (*e.g.*, saline). The terms "therapeutic agent" and "drug" are used interchangeably herein.

The term "therapeutically effective," as used herein in the context of a therapeutic agent, refers to an amount of a therapeutic agent that is sufficient to attain a desired effect.

The term "proximal end" (or "first end") is used herein in connection with components and/or structures which are closer to a clinician or other individual who is using a drug delivery system of the invention in a medical treatment setting. Conversely, the term "distal end" (or "second end") is used herein in connection with components and/or structures which are closer to the treatment site or sampling site within the body of the subject being treated.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a device" includes a plurality of such devices and reference to "the delivery method" includes reference to one or more methods and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION OF THE INVENTION

### Overview

The present invention provides an inner ear delivery device for delivering fluids into the inner ear of a mammalian subject. The device generally comprises a delivery body, which defines a delivery lumen and an overflow lumen; and a delivery tip. The delivery lumen and the overflow lumen terminate at their distal ends in the delivery tip, e.g., the delivery lumen and the overflow lumen are in fluid communication with the delivery tip. The proximal end of the delivery body is adapted to receive a fluid. Fluid introduced into the delivery body flows through the delivery lumen and into the drug delivery tip. The delivery tip is adapted to fit substantially in the round window niche. The delivery tip is adapted to allow a fluid formulation containing a therapeutic agent to exit the device and contact the round window membrane. The therapeutic agent crosses the round window membrane (e.g., by diffusion) and enters the inner ear. The proximal end of the overflow lumen may be co-terminal with the proximal end of the delivery lumen. Alternatively, the proximal end of the overflow lumen may be an outlet in a sidewall of the delivery body. The outlet, which may be a skive, or port, allows excessive fluid to flow from the overflow lumen out of the delivery body at a site other than the round window niche.

A salient feature of the fluid delivery device of the invention is that it is self contained, i.e., once it is implanted into the ear of a subject, it functions independently without the need for attaching the device to a catheter, a pump, or any other components. The fact that the instant device functions in the absence of components that are externally visible, such as catheters, pumps, and the like, increases patient comfort and compliance. Thus, in many embodiments, the device of the invention does not include catheters, pumps, or other externally visible components.

A further salient feature of the delivery device of the invention is that it consistently and reproducible delivers very low volumes of liquid formulation to the inner ear of a subject being treated. The device delivers doses that are therapeutically effective, yet below the ototoxic threshold for the agent being delivered.

### DELIVERY DEVICE MATERIALS AND GENERAL CHARACTERISTICS

The delivery device of the present invention comprises a delivery body and a delivery tip. The delivery body defines a delivery lumen and an overflow lumen, both of which terminate at their distal ends in the delivery tip. The delivery lumen and the overflow lumen are not co-axial; rather, they are in a side-by-side, or parallel, arrangement.

The delivery body is generally a flexible elongate structure comprising a proximal end, a distal end, and an outer surface. The delivery body can be any suitable shape including, but not limited to, tubular, elliptical, cylindrical, etc., and may be either smooth on the delivery body outer surface, or may comprise ridges (e.g., longitudinal, axial, or circumferential) or other surface variations as may be deemed desirable.

The delivery tip is generally spheroid, ovoid, or bulb-like in configuration, or may be irregular in shape, or adapted to fit into an irregularly shaped round window niche. Thus, in some embodiments, the delivery tip is sized for placement in the round window niche of an ear. '

In some embodiments, the delivery tip comprises a solid or semi-solid material that provides for transfer (e.g., capillary transfer) of fluid from the delivery lumen out the delivery tip. Exemplary materials include sponges, or other such materials. The solid or semi-sold material can be covered entirely or partially with a drug-permeable material.

In other embodiments, the delivery tip defines a delivery tip reservoir, and is thus adapted to contain a liquid formulation. Where the delivery tip defines a reservoir, the delivery tip can comprise a porous material, a semipermeable membrane, and the like. Alternatively, the delivery tip can comprise a substantially drug-impermeable material, in which case the delivery tip comprises a plurality of pores or fenestrations, to allow a liquid formulation to pass through. In some embodiments, the entire delivery tip comprises pores or fenestrations. In other embodiments, a portion of the delivery tip comprises pores or fenestrations, e.g., from 5% to about 10%, from about 10% to about 20%, from about 20% to about 30%, from about 30% to about 40%, from about 40% to about 50%, from about 50% to about 60%, from about 60% to about 70%, from about 70% to about 80%, or from about 80% to about 90%, of the delivery tip comprises pores or fenestrations. Where the delivery tip defines a delivery tip reservoir, the delivery lumen and the overflow lumen terminate within the delivery tip reservoir.

### Dimensions

In general, the dimensions of the delivery body (e.g., overall length, outer diameter, inner diameter, wall thickness, etc.) can be varied as required or desired, and will vary according to a variety of factors. In general, the overall length of the delivery body is from about 0.5 cm to about 4 cm, from about 0.75 cm to about 3.5 cm, or from about 1 cm to about 3 cm.

The total capacity of the delivery device is from about 1 µl to about 50 µl, e.g., from about 1 µl to about 3 µl, from about 3 µl to about 5 µl, from about 5 µl to about 10 µl, from about 10 µl to about 15 µl, from about 15 µl to about 20 µl, from about 18 µl to about 22 µl, from about 20 µl to about 25 µl, from about 25 µl to about 30 µl, from about 30 µl to about 35 µl, from about 40 µl to about 45 µl, or from about 45 µl to about 50 µl.

In general, the device of the invention can be described as comprising a delivery body and a delivery tip. The delivery body defines a delivery lumen and an overflow lumen. The delivery lumen extends the length of the delivery body, from the proximal end to the distal end, and terminating in the delivery tip. At its proximal end, the delivery lumen receives liquid, the liquid flows through the delivery lumen, and into the delivery tip.

The overflow lumen extends at its proximal end from an outlet in a sidewall of the delivery body, and terminates in the delivery tip. The overflow lumen may extend the entire length of the delivery body. The overflow lumen outlet can be provided in any configuration, e.g., a port, a plurality of pores, a skive, and the like.

Alternatively, the outlet which is the proximal end of the overflow lumen may be positioned at a distance of from about 0.1 mm to about 250 mm, e.g., from about 0.1 mm to about 1 mm, from about 1 mm to about 10 mm, from about 10 mm to about 50 mm, from about 50 mm to about 100 mm, from about 100 mm to about 150 mm, from about 150 mm to about 200 mm, or from about 200 mm to about 250 mm, or more, from the proximal end of the delivery body. The outlet is typically at least about 10 mm, at least about 50 mm, at least about 100 mm, at least about 0.5 cm, at least about 1 cm, at least about 1.5 cm, at least about 2 cm, or more, away from the distal end of the delivery body or from the delivery tip. In these embodiments, the delivery body comprises a proximal, single-lumen portion comprising only the delivery lumen; and a distal, double-lumen portion comprising both the delivery lumen and the overflow lumen.

The delivery body outer diameter can be substantially the same throughout its length, or can be varied (e.g., tapered, greater at the proximal end than at the distal end, etc.). In one exemplary embodiment, the outer diameter of the delivery body changes abruptly just before the overflow lumen outlet such that the section of the delivery body between the proximal end and just before the overflow lumen outlet has a larger outer diameter than the section of the delivery body between the overflow lumen outlet and the distal end of the delivery body.

In exemplary, non-limiting embodiments, the outer diameter of the proximal section of the delivery body (the single-lumen section) is generally from about 0.5 mm to about 3 mm, from about 0.75 mm to about 2.75 mm, from about 1 mm to about 2.5 mm, and in some embodiments is about 2 mm. The outer diameter of the distal, dual-lumen section of the delivery body is usually from about 0.5 mm to about 3 mm, from about 0.75 mm to about 2.75, from about 1 mm to about 2.5 mm, and in some embodiments is about 1 mm.

The proximal end of the delivery body proximal portion is adapted to receive liquid. In one non-limiting exemplary embodiment, the proximal end of the delivery body is fitted with an injection port. The injection port may comprise a self-sealing element. e.g., a diaphragm, an iris-type closure, or a septum.

The delivery device terminates in a delivery tip. The delivery tip is generally adapted for, e.g., sized for, positioning or placement of the delivery tip within the round window niche of the ear. The diameter of the delivery tip is generally from 1 mm to about 5 mm, from about 1.5 mm to about 4 mm, from about 2 mm to about 3 mm. In particular embodiments, the diameter of the delivery tip is about 1.5 mm, about 2.0 mm, or 2.5 mm. In some embodiments, the delivery tip is sized such that it at least partially engages (has contact with) an internal sidewall of the round window niche. In some embodiments, the delivery tip is sized such that it completely fills the entrance to the round window niche, creating a seal with the entrance to the round window niche, and creating a fluid-receiving zone between the delivery tip and the round window membrane.

### Fluid volumes and dosages of therapeutic agent

The delivery devices of the instant invention are particularly well suited for consistently and reproducibly delivering very low volumes and dosages to the inner ear of a subject.

The subject delivery devices are adapted for delivery of volumes of formulation in the microliter and submicroliter range, e.g., from about 1 µl to about 50 µl, e.g., from about 1 µl to about 3 µl, from about 3 µl to about 5 µl, from about 5 µl to about 10 µl, from about 10 µl to about 15 µl, from about 15 µl to about 20 µl, from about 18 µl to about 22 µl, from about 20 µl to about 25 µl, from about 25 µl to about 30 µl, from about 30 µl to about 35 µl, from about 40 µl to about 45 µl, or from about 45 µl to about 50 µl.

The subject delivery devices are adapted for delivery at low volume rates. For example, a delivery device of the invention delivers liquid formulation to an inner ear of a subject at a rate of from about 0.01 µl/hour to about 5 µl/hour, e.g, from about 1 µl/hour to about 5 µl/hour, from about 0.05 µl/hour to about 1 µl/hour, or from about 0.01 µl/hour to about 0.05 µl/hour.

Total single dosages of therapeutic agent are large enough to be therapeutically effective, yet lower than the ototoxic threshold for the particular therapeutic agent. A single dose is considered the amount of therapeutic agent that is delivered in the total maximum volume of the delivery device, e.g., where the total volume of the device is about 20 µl, a single dose is the total amount, in µg that is delivered in 20 µl of liquid formulation. Those skilled in the art can readily determine the ototoxic threshold for any given therapeutic agent. As one non-limiting example, gentamycin is delivered at from about 100 µg to about 500 µg, from about 125 µg to about 450 µg. from about 150 µg to about 400 µg, from about 150 µg to about 350 µg, or from about 200 µg to about 300 µg in a single dose.

### Delivery device materials

The delivery device comprises a biocompatible material, more preferably an implantable grade biocompatible material. The material of the delivery device is generally substantially drug-impermeable (with the possible exception of the delivery tip, which may comprise a porous or semi-permeable material) and comprises a material(s) that does not react in an unintended manner with the active agent formulation. The delivery device can be made of a single material, or can comprise two or more materials layered upon one another.

Exemplary materials include, but are not necessarily limited to, biocompatible polymers, bioerodable materials, elastomers, metals, metal alloys, glasses, laminates of hydrophilic polymers and hydrophobic polymers, multilaminates or polymer, metals, and/or glasses; and the like.

Specific exemplary biocompatible polymeric materials include, but are not necessarily limited to, homopolymers and copolymers of vinyl acetate (e.g., ethylene vinyl acetate copolymer); homopolymers and copolymers of acrylates (e.g., poly(methyl) methacrylate (PMMA), polyethylmethacrylate, ethylene glycol dimethacrylate, ethylene dimethacrylate and hydroxymethyl methacrylate); polyurethanes; polyethylenes; polyvinylchlorides; polycarbonates; polyamides; polysulfones; polyesters; polyimides; halogenated polymers (e.g., polytetrafluoroethylene (PTFE), polyvinyl fluoride, polychlorotrifluoroethylene, copolymers tetrafiuoroethylene and hexafluoropropylene; PFA, and the like); polyolefins (e.g., high density polyethylene (HDPE), low density polyethylene (LDPE), linear low density polyethylene (LLDPE), polypropylenes, and the like); polystyrenes; nylons; urethanes; homopolymers and copolymers of acrylonitrile (e.g., acrylonitrile-butadiene-styrene polymer, styrene acrylonitrile, polycarbonate-acrylonitrile-butadiene-styrene; and the like); polyvinylpyrrolidone; 2-pyrrolidone; polyacrylonitrile butadiene; cellulose acetate; polyethylene terephtholate; polymethylpentene; polyisobutylene; polymethylstyrene; polyvinylidine chloride and homopolymers and copolymers of polyvinylidine chloride (e.g., polyvinylchloride-acrylic copolymers); PEBAX^{™}; HYTREL^{™}; and other similar compounds known to those skilled in the art. Further exemplary polymers are described in Plastics Materials 6^{th} ed., May 1995, J.A. Brydson, Butterworth-Heinemann, publishers.

Suitable, biocompatible elastomers include, but are not necessarily limited to, biocompatible elastomers such as medical grade synthetic (*e.g.,* silicone) rubbers; polyvinyl chloride elastomers; polyolefins; homopolymeric and copolymeric elastomers: urethane-based elastomers; natural rubbers; and fluorinated polymers (e.g., PTFE), and the like.

Metallic materials suitable for the delivery body comprise stainless steel, titanium, platinum, tantalum, gold and their alloys; gold-plated ferrous alloys; platinum-plated titanium, stainless steel, tantalum, gold and their alloys as well as other ferrous alloys; cobalt-chromium alloys; titanium nitride-coated stainless steel, titanium, platinum, tantalum, gold, and their alloys; TEFLON^{™}; nickel titanium; and superelastic nickel titanium.

The delivery device can comprise additional materials or agents. For example, the delivery body can comprise a coating on an internal wall of the lumen to facilitate transport of liquid through the lumen, or to impart other desirable characteristics to the delivery body. The lumen can also comprise coatings that reduce the risk of infection, *e.g.,* a silver coating, a coating or treatment with an antimicrobial agent(s), etc. The outer surface of the delivery device can comprise a coating or be treated to facilitate implantation of the device within the subject (e.g., a lubricious coating), to reduce the risk of infection, and/or to impart other desirable characteristics to the drug delivery device.

The delivery tip may comprise a substantially drug-impermeable material, as described above. Alternatively, the delivery tip may comprise a porous material, a semi-permeable membrane that selectively allows fluids to pass therethrough. Exemplary semi-permeable membranes suitable for use in the present invention include but are not limited to those which are known in the art and described in Kiil, F., Am J. Physiology, 256-260:(April 1989); Erickson, D., Sci. American, vol. 267(3), pp. 163-164 Satoh, Y. et al., Keio J. Med., vol. 41:(1), pp. 16-22 (March 1992) which are incorporated herein by reference. The semi-permeable membrane may be substituted with a micropore filter known in the art and suitable for the purposes set forth herein. Many such filters are commercially available, e.g., from Millipore, Inc. of Bedford, Mass. (USA).

### Additional components

In some embodiments, a subject delivery device further comprises an element adapted for positioning or securing the device in the round window niche, e.g., to facilitate retention of the device in the round window niche. In some embodiments, the element is disposed around the delivery body at a site immediately adjacent to the delivery tip, and may be attached to the delivery body. In some embodiments, the element provides for contact with the round window niche so as to form a sealable contact with the round window niche. Where the contact is sealable, the element engages an internal sidewall of the round window niche at least partially, and creates a fluid-receiving zone between the element and the round window membrane. In some embodiments, the element is toroidal or annular element, e.g., a bladder. In some embodiments, the element is inflatable, e.g., with a gas or with a fluid. In some embodiments, the element is fixedly attached to the delivery body (e.g., fused to the delivery body); in other embodiments, the element is not fixedly attached, e.g., the element can be readily removed from the delivery body.

### Tissues to be treated

Exemplary inner ear tissue structures of primary importance for treatment purposes include, but are not limited to, the cochlea, the endolymphatic sac/duct, the vestibular labyrinth, and all of the compartments that include these components. Access to the above-described inner ear tissue regions is typically achieved through a variety of structures, including but not limited to the round window membrane, the oval window/stapes footplate, the annular ligament, the otic capsule/temporal bone, and the endolymphatic sac/endolymphatic duct, all of which shall be considered middle-inner ear interface tissue structures as described in greater detail below. In addition, as indicated herein, the middle ear shall be defined as the physiological air-containing tissue zone behind the tympanic membrane (e.g. the ear drum) and ahead of the inner ear.

### DRUG DELIVERY METHODS

The device disclosed herein is usable in methods of delivering a therapeutic agent (also referred to herein as a "drug") to an inner ear of a mammalian subject. The methods generally involve introducing a device of the invention into the ear of a subject to be treated. A liquid formulation in the device flows from the delivery tip and into the round window niche. The therapeutic agent then diffuses across the round window membrane into the inner ear.

Thus, the device disclosed herein is usable in a method for delivery of a therapeutic agent to an inner ear of a subject, the method generally involving implanting a delivery device of the invention into a round window niche of a subject, where the implanted device provides a delivery pathway from the proximal end of the device, through the delivery lumen, and out the delivery tip into the window niche in a subject.

In some embodiments, the delivery device is loaded with liquid formulation prior to implantation. In these embodiments, the delivery lumen is adapted to contain a liquid formulation comprising a therapeutic agent, thereby serving as a reservoir. In other embodiments, the device is first implanted, and then liquid formulation is introduced into the device. When the device is filled with liquid formulation after implantation a conventional syringe/needle assembly is typically used, wherein the needle is inserted into the external auditory canal of the subject, and into the proximal end of the delivery body, e.g., through a septum. If the amount of liquid injected into the device exceeds the total capacity of the device, excess liquid enters the overflow lumen and exits the overflow lumen outlet and into the outer ear.

The delivery device can be designed for temporary use, or to remain implanted in the subject for an extended period, e.g., from several days, to several weeks or months, and can be designed to be substantially permanently implanted in the subject (e.g., for the subject's remaining lifespan). In other embodiments, the device remains in the subject for a period of from about 3 hours to about 6 hours, from about 6 hours to about 12 hours, from about 12 hours to about 24 hours, from about 24 hours to about 2 days, from about 2 days to about 4 days, from about 4 days to about 7 days, from about 1 week to about 2 weeks, from about 2 weeks to about 4 weeks, or from about 1 month to about 3 months or longer. In some embodiments, where the delivery device is made entirely of a bioerodable material, the delivery device is implanted, and remains in the body of the individual until the device is completely biodegraded in the body of the individual.

The device can be used in a single dose treatment. Alternatively, the implanted device can be accessed by means of a needle/syringe assembly, and additional liquid formulation added. Subsequent introduction of additional liquid formulation is of interest where two or more different therapeutic agents are to be administered, one after the other, or multiple doses of the same therapeutic agent are to be administered. Subsequent doses can be administered immediately following the initial dose is delivered, or can be administered hours, days, weeks, or even months after the initial dose.

The devices of the present invention are preferably rendered sterile prior to use. This may be accomplished by separately sterilizing each component, e.g., by gamma radiation, steam sterilization or sterile filtration, etc., then aseptically assembling the final system. Alternatively, the devices may be assembled, then terminally sterilized using any appropriate method.

### Placement of the device within the ear

The primary treatment apparatus with the insert member therein is then surgically inserted and positioned within the middle ear of a patient so that the reservoir portion of the primary treatment apparatus is in direct physical contact with a selected middle-inner ear interface tissue structure. Surgical insertion and placement in this manner is normally accomplished via an incision in the tympanic membrane which is undertaken using standard tympanotomy procedures. Alternatively, insertion and placement of the apparatus may be accomplished using a standard tympanomeatal flap incision which likewise provides access to the middle ear and structures thereof. An exemplary and preferred middie/inner ear tissue structure suitable for the purposes set forth herein is the round window membrane. Since the device need not be accessed by a catheter, etc, the device may, but does not necessarily, extend outward from the tympanic membrane.

In order to use the foregoing alternative embodiment of the apparatus described above, it is surgically inserted within the middle ear (e.g. so that the reservoir portion is entirely positioned within the middle ear). Surgical insertion in this manner is preferably accomplished through an incision in the tympanic membrane using conventional surgical tympanotomy procedures or alternatively through the use of a tympanomeatal flap procedure as described above. Thereafter, using standard microsurgical techniques, the first stem portion is inserted through a previously selected middle-inner ear interface tissue structure. In a preferred embodiment, the first stem portion is positioned through a discrete opening formed through the stapes footplate (and underlying oval window) or through the cochlear/vestibular otic capsule bone. This opening (formed using laser energy or microdrill techniques) provides access from the middle ear and/or mastoid space into any or all of the various inner ear compartments for the direct placement of the first stem portion therein. As a result, the open first end of the first stem portion is positioned adjacent to and in direct contact with the inner ear fluids (e.g. endolymph and/or perilymph), tissues, compartments, and/or tissue regions to be treated. It should also be noted that the body portion of the foregoing alternative medicine delivery apparatus is suitably positioned so that at least a section of the second stem portion (e.g. the open first end thereof) passes through the incised tympanic membrane (or beneath the foregoing tympanomeatal flap), and resides within the external auditory canal of the ear.

### Therapeutic agents

A wide variety of liquid medicines/therapeutic agents may be used in connection with the apparatus, including but not limited to urea, mannitol, sorbitol, sodium chloride, steroids, heparin, hyaluronidase, aminoglycoside antibiotics (streptomycin/gentamycin), glycerol, xylocaine, immunoglobulins, and other antibiotic, biologically active, or antimicrobial materials. Representative medicines which are typically used to treat inner ear tissues include but are not limited to aminoglycoside antibiotics such as gentamycin and streptomycin, urea, mannitol, sorbitol, glycerol, lidocaine, xylocaine, epinephrine, immunoglobulins, sodium chloride, steroids, heparin, hyaluronidase, antioxidants, neurotrophins, nerve growth factors, various therapeutic peptides, and polysaccharides.

Furthermore, the delivery tip may be initially supplied with solid (e.g. crystalline), gel-type, viscous liquid, or powdered precursor medicine materials which can be hydrated/solvated in situ in order to produce liquid medicine materials. Exemplary solid or gel-type/viscous medicine materials to which water may be added in this manner include but are not limited to mannitol crystals, sodium chloride crystals, viscous liquid glycerol, powdered streptomycin/gentamycin, hyaluronidase gel and the like. Accordingly, the present invention shall not be limited to the delivery of any specific chemical materials, biological compositions, pharmaceuticals, or other therapeutic agents.

### Disorders amenable to treatment

The device disclosed herein is usable in methods for treating an inner ear disorder. The methods generally involve implanting a device of the present invention into a round window niche of a subject, wherein a drug flows from the device to the inner ear of the individual, and the disorder is treated.

Disorders amenable to treatment using the device of the present invention include, but are not limited to, Meniere's disease, autoimmune inner ear disease, allergic inner ear disease, tinnitus, labarynthitis and various forms of hearing loss such as sudden viral deafness, and the like. Whether a particular disorder is treated using a method of the invention can be readily determined by those skilled in the field of otolaryngology, using standard methods.

### EXEMPLARY SPECIFIC EMBODIMENTS OF THE DELIVERY DEVICE OF THE INVENTION

Referring generally to one non-limiting embodiment of a delivery device of the invention illustrated in Figure 1, delivery device **5** of the invention comprises a delivery body **10** which comprises a proximal section **20,** and a distal section **30;** and a delivery tip **40.** In this embodiment, the proximal end **11** of the delivery body **10** is fitted with a septum that serves as an injection port **50**.

Proximal section **20** defines a delivery lumen **13.** Distal section 30 defines, in addition to a delivery lumen **13,** an overflow lumen **14.** Delivery lumen **13** terminates in delivery tip **40.** Overflow lumen **14** extends from outlet 60 to delivery tip **40** and allows excessive liquid introduced by injection to exit the device in a region of the ear proximal to the round window niche.

Delivery tip **40** comprises pores or fenestrations **41** through which liquid formulation flows out into the round window niche **1.** Liquid formulation containing a therapeutic agent diffuses across round window membrane **3** and into the inner ear.

In operation, delivery device **5** is implanted into the ear of the subject being treated such that delivery tip **40** is substantially fitted into the round window niche **1** of the subject. Liquid formulation is introduced into the proximal end **11** of delivery body, e.g., by injection of liquid into injection port **50.** Alternatively, the device is loaded with liquid formulation before being implanted. Liquid formulation flows through delivery lumen **13** and into delivery tip **40.** In the event that the amount of liquid injected into the implanted device exceeds the total capacity of the device, excess liquid flows from delivery tip **40** into overflow lumen **14** and out of outlet **60.** In the exemplary embodiment depicted in Figure 1, liquid formulation flows through fenestrations **41** in delivery tip **40** and into a fluid-receiving zone **2** formed between the delivery tip and the round window membrane **3.**

## Claims

1. A self-contained device (5) for delivering a fluid to the inner ear of a subject, the device comprising:
(a) an elongate delivery body (10) having a proximal end (20) and a distal end (30), which delivery body (5) defines a delivery lumen (13) having a proximal end and a distal end; and
(b) a delivery tip (40) at the distal end of the delivery body,
**characterized in that** said delivery tip comprises an overflow lumen (14) having a proximal end and a distal end, wherein the proximal end of the overflow lumen forms an outlet (60), wherein the distal end of the delivery lumen (13) and the distal end of the overflow lumen (14) are in fluid communication with the delivery tip (40) and wherein the delivery tip (40) defines a reservoir for fluid delivered from the delivery lumen (13) and overflow lumen (14).

2. The device of claim 1, wherein the delivery tip (40) is adapted to fit substantially in the round window niche of the ear.

3. The device of claim 1, wherein the delivery tip (40) is adapted to completely fill the entrance to the round window niche, thereby creating a seal with the entrance to the round window niche, and creating a fluid-receiving zone between the delivery tip (40) and the round window membrane of the ear.

4. The device of any one of claims 1-3, wherein the delivery tip (40) is from about 1 mm to about 5 mm in diameter.

5. The device of any one of claims 1 - 4, wherein the proximal end of the overflow lumen (14) is substantially co-terminal with the proximal end of the delivery lumen(13).

6. The device of any one of claims 1 - 4, wherein the proximal end of the overflow lumen (14) is placed between the proximal and distal ends of the elongate delivery body (10).

7. The device of any one of claims 1 - 6, wherein the proximal end of the overflow lumen (14) comprises a plurality of pores (41).

8. The device of any one of claims 1 - 7 wherein the delivery body (10) has an overall length of from about 0.5cm to about 4cm.

9. The device of any one of claims 1 - 8, wherein the delivery reservoir of the tip (40) contains a drug formulation.

10. The device of any one of claims 1 - 9, wherein the device (5) has a fluid capacity of from about 1 µl to about 50 µl.

11. The device of any one of claims 1 - 10, wherein the proximal end (20) of the delivery body (10) is fitted with an injection port (50).

12. The device of claim 11, wherein the injection port (50) comprises a self-sealing element.

13. The device of any one of claims 1 - 8 or 10 - 12, further comprising a liquid formulation, the formulation comprising a therapeutic agent.

14. The device of any one of claims 1 - 13, wherein the device is adapted for delivery of a liquid formulation to an inner ear of a subject at a rate of about 1 µl/hour to about 5 µl/hour.

15. The device of any one of claims 1 - 14, wherein the device is loaded with a liquid formulation prior to implantation.

16. The device of any one of claims 1 - 14, wherein the device is adapted to be loaded with a liquid formulation after implantation.

## Patentansprüche

1. Unabhängige Vorrichtung (5) für die Abgabe eines Fluids an das Innenohr einer Person, wobei die Vorrichtung folgendes umfasst:
(a) einen elongierten Abgabekörper (10) mit einem proximalen Ende (20) und einem distalen Ende (30), wobei der Abgabekörper (5) ein Abgabelumen (13) mit einem proximalen Ende und mit einem distalen Ende definiert; und
(b) eine Abgabespitze (40) an dem distalen Ende des Abgabekörpers;
**dadurch gekennzeichnet, dass** die genannte Abgabespitze ein Überlauflumen (14) mit einem proximalen Ende und einem distalen Ende aufweist, wobei das proximale Ende des Überlauflumen einen Auslass (60) bildet, wobei das sich distale Ende des Abgabelumen (13) und das distale Ende des Überlauflumen (14) in Fluidübertragungsverbindung mit der Abgabespitze (40) befinden, und wobei die Abgabespitze (40) einen Speicher für Fluid definiert, das von dem Abgabelumen (13) und dem Überlauflumen (14) abgegeben wird.

2. Vorrichtung nach Anspruch 1, wobei die Abgabespitze (40) im Wesentlichen um die runde Fensternische des Ohrs passt.

3. Vorrichtung nach Anspruch 1, wobei die Abgabespitze (40) den Eingang in de runde Fensternische vollständig füllen kann, wodurch ein dichter Verschluss mit dem Eingang in die runde Fensternische erzeugt wird, und wobei eine Fluidaufnahmezone zwischen der Abgabespitze (40) und der runden Fenstermembran des Ohres erzeugt wird.

4. Vorrichtung nach einem der Ansprüche 1 bis 3, wobei die Abgabespitze (40) einen Durchmesser zwischen etwa 1 mm und etwa 5 mm aufweist.

5. Vorrichtung nach einem der Ansprüche 1 bis 4, wobei das proximale Ende des Überlauflumen (14) im Wesentlichen gemeinsam mit dem proximalen Ende des Abgabelumen (13) endet.

6. Vorrichtung nach einem der Ansprüche 1 bis 4, wobei das proximale Ende des Überlauflumen (14) zwischen den proximalen und distalen Enden des elongierten Abgabekörpers (10) platziert ist.

7. Vorrichtung nach einem der Ansprüche 1 bis 6, wobei das proximale Ende des Überlauflumen (14) eine Mehrzahl von Poren (41) umfasst.

8. Vorrichtung nach einem der Ansprüche 1 bis 7, wobei der Abgabekörper (0) eine Gesamtlänge zwischen etwa 0,5 cm und etwa 4 cm aufweist.

9. Vorrichtung nach einem der Ansprüche 1 bis 8, wobei die Abgabespitze (40) eine Arzneimittelzusammensetzung aufweist.

10. Vorrichtung nach einem der Ansprüche 1 bis 9, wobei die Vorrichtung (5) eine Fluidkapazität zwischen etwa 1 µl und etwa 50 µl aufweist.

11. Vorrichtung nach einem der Ansprüche 1 bis 10, wobei das proximale Ende (20) des Abgabekörpers (10) mit einem Injektionsanschluss (50) versehen ist.

12. Vorrichtung nach Anspruch 11, wobei der Injektionsanschluss (50) ein selbst abdichtendes Element umfasst.

13. Vorrichtung nach einem der Ansprüche 1 bis 8 oder 10 bis 12, wobei diese ferner eine flüssige Zusammensetzung umfasst, wobei es sich bei der Zusammensetzung um ein Therapeutikum handelt.

14. Vorrichtung nach einem der Ansprüche 1 bis 13, wobei die Vorrichtung eine flüssige Zusammensetzung an ein Innenohr einer Person mit einer Rate von etwa 1 µl/Stunde bis etwa 5 µl/Stunde abgeben kann.

15. Vorrichtung nach einem der Ansprüche 1 bis 14, wobei die Vorrichtung vor der Implantation mit einer flüssigen Zusammensetzung beschickt wird.

16. Vorrichtung nach einem der Ansprüche 1 bis 14, wobei die Vorrichtung nach der Implantation mit einer flüssigen Zusammensetzung beschickt werden kann.

## Revendications

1. Dispositif autonome (5) pour administrer un fluide dans l'oreille interne d'un sujet, le dispositif comprenant :
(a) un corps d'administration allongé (10) ayant une extrémité proximale (20) et une extrémité distale (30), lequel corps d'administration (5) définit une lumière d'administration (13) ayant une extrémité proximale et une extrémité distale ; et
(b) un embout d'administration (40) au niveau de l'extrémité distale du corps d'administration, **caractérisé en ce que** ledit embout d'administration comprend une lumière de déversement (14) ayant une extrémité proximale et une extrémité distale, dans lequel l'extrémité proximale de la lumière de déversement forme une sortie (60) dans lequel l'extrémité distale de la lumière d'administration (13) et l'extrémité distale de la lumière de déversement (14) sont en communication de fluide avec l'embout d'administration (40) et dans lequel l'embout d'administration (40) définit un réservoir pour le fluide délivré à partir de la lumière d'administration (13) et de la lumière de déversement (14).

2. Dispositif selon la revendication 1, dans lequel l'embout d'administration (40) est adapté pour se monter sensiblement dans la fenêtre ronde de l'oreille.

3. Dispositif selon la revendication 1, dans lequel l'embout d'administration (40) est adapté pour remplir complètement l'entrée dans la fenêtre ronde, créant ainsi un joint entre l'entrée dans la fenêtre ronde, et créant une zone de réception de fluide entre l'embout d'administration (40) et la fenêtre ronde de l'oreille.

4. Dispositif selon l'une quelconque des revendications 1 à 3, dans lequel l'embout d'administration (40) mesure d'environ 1 mm à environ 5 mm de diamètre.

5. Dispositif selon l'une quelconque des revendications 1 à 4, dans lequel l'extrémité proximale de la lumière de déversement (14) se termine sensiblement de manière conjointe avec l'extrémité proximale de la lumière d'administration (13).

6. Dispositif selon l'une quelconque des revendications 1 à 4, dans lequel l'extrémité proximale de la lumière de déversement (14) est placée entre les extrémités proximale et distale du corps d'administration allongé (10).

7. Dispositif selon l'une quelconque des revendications 1 à 6, dans lequel l'extrémité proximale de la lumière de déversement (14) comprend une pluralité de pores (41).

8. Dispositif selon l'une quelconque des revendications 1 à 7, dans lequel le corps d'administration (10) a une longueur globale de l'ordre d'environ 0,5 cm à environ 4 cm.

9. Dispositif selon l'une quelconque des revendications 1 à 8, dans lequel le réservoir d'administration de l'embout (40) contient une formule médicamenteuse.

10. Dispositif selon l'une quelconque des revendications 1 à 9, dans lequel le dispositif (5) a une capacité de liquide de l'ordre d'environ 1 µl à environ 50 µl.

11. Dispositif selon l'une quelconque des revendications 1 à 10, dans lequel l'extrémité proximale (20) du corps d'administration (10) est montée avec un orifice d'injection (50).

12. Dispositif selon la revendication 11, dans lequel l'orifice d'injection (50) comprend un élément auto-obturateur.

13. Dispositif selon l'une quelconque des revendications 1 à 8 ou 10 à 12, comprenant en outre une formule liquide, la formule comprenant un agent thérapeutique.

14. Dispositif selon l'une quelconque des revendications 1 à 13, dans lequel le dispositif est adapté pour l'administration d'une formule liquide dans une oreille interne d'un sujet à une vitesse d'environ 1 µl/heure à environ 5 µl/heure.

15. Dispositif selon l'une quelconque des revendications 1 à 14, dans lequel le dispositif est chargé avec une formule liquide avant l'implantation.

16. Dispositif selon l'une quelconque des revendications 1 à 14, dans lequel le dispositif est adapté pour être chargé avec une formule liquide après l'implantation.
